# EUROPEAN PATENT SPECIFICATION

(11) **EP 4 574 182 B1**
(45) Date of publication and mention of the grant of the patent: **26.08.2026**
(21) Application number: 25167485.9
(22) Date of filing: 27.10.2020
(51) Int. Cl.: A61L 29/08, A61L 29/16

(54) **URINARY CATHETERS AND METHODS FOR PREVENTING BACTERIAL INFECTIONS**
HARNKATHETER UND VERFAHREN ZUR VERHINDERUNG BAKTERIELLER INFEKTIONEN
CATHÉTERS URINAIRES ET PROCÉDÉS DE PRÉVENTION D'INFECTIONS BACTÉRIENNES

(30) Priority: 28.10.2019 US 201962926923 P
(43) Date of publication of application: 25.06.2025
(62) Document number(s) of the earlier application(s) in accordance with Art. 76 EPC: 20811182.3 / 4 051 330
(73) Proprietor: Hollister Incorporated, Libertyville, IL 60048-3781 (US)
(72) Inventor: SILEIKA, Tadas S., Libertyville (US); DEAN, Nicole L., Libertyville (US); PETTIGREW, Jacqueline MJ, Libertyville (US)
(74) Representative: FRKelly

(56) References cited:
- WO-A1-2016/146806
- ASADISHAD BAHAREH ET AL: "Pomegranate materials inhibit flagellin gene expression and flagellar-propelled motility of uropathogenic Escherichia coli strain CFT073", vol. 334, no. 2, 12 July 2012 (2012-07-12), pages 87 - 94, XP055772793, ISSN: 0378-1097, Retrieved from the Internet <URL:https://academic.oup.com/femsle/article-pdf/334/2/87/19120863/334-2-87.pdf> DOI: 10.1111/j.1574-6968.2012.02622.x
- CHAN MICHELLE ET AL: "Inhibition of bacterial motility and spreading via release of cranberry derived materials from silicone substrates", COLLOIDS AND SURFACES B: BIOINTERFACES, ELSEVIER AMSTERDAM, NL, vol. 110, 29 April 2013 (2013-04-29), pages 275 - 280, XP028573101, ISSN: 0927-7765, DOI: 10.1016/J.COLSURFB.2013.03.047
- RÜTSCHLIN SINA ET AL: "Inhibitors of Bacterial Swarming Behavior", vol. 26, no. 5, 24 January 2019 (2019-01-24), DE, pages 964 - 979, XP055772894, ISSN: 0947-6539, Retrieved from the Internet <URL:https://onlinelibrary.wiley.com/doi/full-xml/10.1002/chem.201901961> DOI: 10.1002/chem.201901961

## Description

The present application claims the benefit of and priority to U.S. Provisional Patent Application No. 62/926,923, filed October 28, 2019.

### DESCRIPTION

### TECHNICAL FIELD

The present disclosure generally relates to urinary catheters and methods for preventing bacterial infections, and more particularly, to catheters and methods for preventing urinary tract infections. The present disclosure also relates to urinary catheters and methods that hinder the motility structures of bacteria within the urethra.

### BACKGROUND

It is desirable for urinary catheters to have a lubricated or lubricious outer surface to increase comfort of the patient during insertion into and/or removal from the body. One method for rendering the surface of a urinary catheter lubricious is to coat at least the insertable portion of the catheter with a lubricious hydrophilic coating. Another method is to apply a lubricant to the urinary catheter. Lubricity of the catheter minimizes soft tissue damage and reduces overall discomfort during use of the medical device.

Although catheters are typically prepared in sterile environments and are provided with safe handling instructions, catheter users are at risk of contracting a urinary tract infection due to several different factors. For example, the catheter may become contaminated during use and introduce bacteria into the urethra. The catheter also may carrier bacteria along the urinary tract. Additionally, because urine is voided from the bladder through the catheter, urine does not flow directly through the urethra. Thus, urine is no longer a factor in flushing out the urethra or wetting of the urethral tissue.

Therefore, there is a need for catheters which reduce the risk of urinary tract infections.

CHAN MICHELLE ET AL: "Inhibition of bacterial motility and spreading via release of cranberry derived materials from silicone substrates", COLLOIDS AND SURFACES B: BIOINTERFACES, ELSEVIER AMSTERDAM, NL, vol. 110, 29 April 2013, pages 275-280, ISSN: 0927-7765, DOI: 10.1016/J.COLSURFB.2013.03.047 teaches that cranberry derived materials (CDMs) impair bacterial motility and that their incorporation into and release from catheters may yield considerable benefits to patient health.

### SUMMARY

The scope of protection is defined by the claims.

The invention relates to a urinary catheter product according to claims 1-7.

### BREIF DESCRIPTION OF THE DRAWINGS

Fig. 1 is a plan view of one embodiment of the present disclosure; and
Fig. 2 is a plan view of one embodiment of a catheter of the present disclosure.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

For catheter users, whether in-dwelling or intermittent, urine is no longer a factor that participates in regular flushing or wetting of the urethra. Because the users are unable to void directly through the urethra, the urethra no longer experiences the continuous flushing and re-wetting of the tissues that normally occurs during active voluntary urination. When the mechanism fails, the risk of urinary tract infections increases.

Urinary tract infections can be a result of bacteria swarming and/or traveling up through the urethra towards the bladder. Traveling of bacteria up the urethra is typically not a concern for those who can perform voluntary urination, because normal voiding tends to wash out and eliminate the bacteria from urethra, thereby prohibiting it from traveling toward the bladder and causing infection.

There are certain species of bacteria that are considered good "swimmers," which swarm and move when they receive certain signals from the surrounding environment. These bacteria are capable of movement even on solid surfaces through excretion of their own extracellular fluids. These motile bacteria also are known to become more pathogenic and increase their motility organelles, such as flagella (a process called hyperflagellation), when sensing certain signals, such as decreased viscosity in the fluids and mucus in their surrounding environment and/or an increase in chemotactants.

In a non-voiding patient, the urethral environment will be fairly viscous, due to the presence of mucosal secretions. When a hydrophilic catheter or a catheter lubricated with a water-based gel is introduced into the urethra, such introduction will likely deposit additional moisture, diluting the secretions, thus decreasing the overall viscosity of the urethral mucus and the surrounding urethral environment. This decrease in the urethral mucus viscosity provides a signal that activates the motility of the bacteria.

Furthermore, when a catheter is inserted into the urethra, the catheter, hydrophilic coating, hydration medium and/or lubricants may include a chemotactant that promotes chemotaxis (the movement of the bacteria in response to a chemical stimulant). For example, the hydrophilic coating and hydration medium may include a chemotactant, such as a polyol or polysaccharide, which stimulates the bacteria to move toward the chemotactant. If the catheter distributes this chemotactant at or near the bladder, the bacteria may be stimulated to undesirably swarm and move toward the bladder.

The catheter products disclosed herein target and hinder the mobility structures of the bacteria, such as the flagella and pili of the bacteria. The flagella and pili are some of the structures of the bacteria that are responsible for mobility. The catheter products of the present disclosure include agents that target and hinder, inhibit or disable the flagella and/or pili from performing their mobility functions. Hindering the flagella and/or pili from performing their functions, mitigates the risk of bacteria swarming and traveling proximally up the urethra toward the bladder. Thus, when bacteria are stimulated to swarm and move by signals from changes in the urethral environment, the bacteria do not effectively move and/or swarm because the agent has hindered the motility structure of the bacteria. The agents may be incorporated into the catheter product in a manner that the agents are delivered and/or distributed into the urethra by the catheter product.

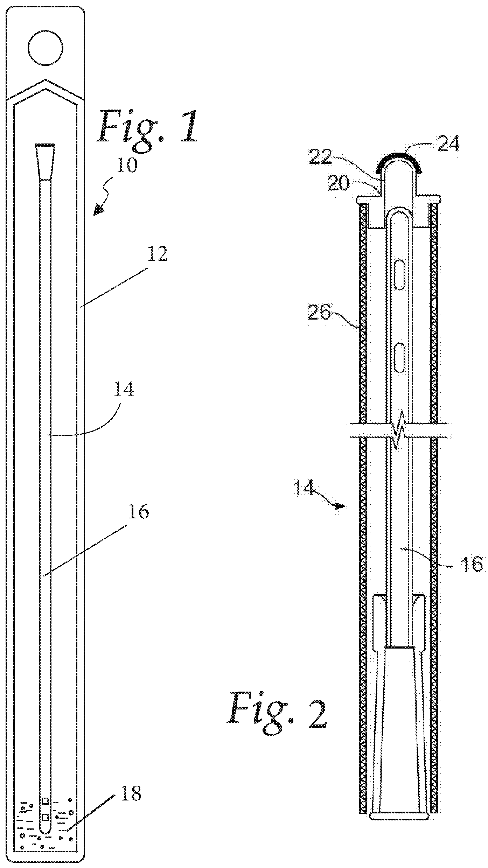

The agent that inhibits motility structures of bacteria is included in in the hydrophilic coating. For example, referring to Fig. 1, a catheter product 10 includes a package 12 including a catheter 14 having a catheter shaft 16. The outer surface of the catheter shaft 16 is hydrophilic. The catheter 16 is coated with a hydrophilic coating. The hydrophilic surface of the catheter shaft 16 becomes lubricious when wetted with a hydration medium 18 (water or saline). The agent that inhibits motility structures of the bacteria is contained in the hydrophilic coating and optionally in the hydration medium. When the catheter product includes a lubricating gel, the compound may be included in the lubricating gel. When the catheter is inserted into and through the urethra, the agent may be distributed along the urethra, where it will come into contact with bacteria.

Referring to Fig. 2, when the catheter product includes an introducer aid 20 that may be inserted into the urethral opening and which the catheter is inserted through, the introducer aid 20 or the tip 22 thereof may include the agent that inhibits motility structures of bacteria 24. When the catheter shaft 16 is inserted through the introducer aid 20, the agent is disposed onto the catheter shaft 16 wherein the catheter distributes the agent along the urethra. Additionally, a sleeve 26 may be attached to the introducer aid, wherein the sleeve surrounds the catheter.

The agent includes punicalagin which targets and inhibits flagella of bacteria, thereby inhibiting the motility and swarming of the bacteria.

## Claims

1. A urinary catheter product, comprising:
a catheter having a catheter shaft and a hydrophilic coating on the catheter shaft, wherein the hydrophilic coating becomes lubricious when wetted; and
wherein the hydrophilic coating comprises an agent that hinders the motility structures of bacteria wherein said agent comprises punicalagin;
wherein the catheter product further includes a hydration medium for hydrating the hydrophilic coating.

2. The urinary catheter product of claim 1, wherein the hydration medium comprises water or saline.

3. The urinary catheter product of claim 1 or 2, wherein the catheter comprises an introducer aid.

4. The urinary catheter product of any one of claims 1-3, wherein a sleeve is attached to the introducer aid, wherein the sleeve surrounds the catheter.

5. The urinary catheter product of any one of claims 1-4, wherein the agent further comprises branched-chain fatty acids, hydroxyindoles, naphthalene derivatives, quinazoline-2,4-diamino analogs, salicylaldehyde hydrazones, salicylidene anilines, and/or thiazolidinones.

6. The urinary catheter product of any one of claims 1-5, wherein the hydration medium further comprises the agent.

7. The urinary catheter product of any one of claims 1-6, further comprising a package.

## Patentansprüche

1. Harnkatheterprodukt, umfassend:
einen Katheter, der einen Katheterschaft und eine hydrophile Beschichtung auf dem Katheterschaft aufweist,
wobei die hydrophile Beschichtung bei Benetzung gleitfähig wird; und
wobei die hydrophile Beschichtung ein Mittel umfasst, das die Beweglichkeitsstrukturen von Bakterien behindert, wobei das Mittel Punicalagin umfasst;
wobei das Katheterprodukt ferner ein Hydratationsmedium zum Hydratisieren der hydrophilen Beschichtung beinhaltet.

2. Harnkatheterprodukt nach Anspruch 1, wobei das Hydratationsmedium Wasser oder Kochsalzlösung umfasst.

3. Harnkatheterprodukt nach Anspruch 1 oder 2, wobei der Katheter eine Einführhilfe umfasst.

4. Harnkatheterprodukt nach einem der Ansprüche 1 bis 3, wobei eine Hülse an der Einführhilfe angebracht ist, wobei die Hülse den Katheter umgibt.

5. Harnkatheterprodukt nach einem der Ansprüche 1 bis 4, wobei das Mittel ferner verzweigtkettige Fettsäuren, Hydroxyindole, Naphthalinderivate, Chinazolin-2,4-diamino-Analoga, Salicylaldehydhydrazone, Salicylidenaniline und/oder Thiazolidinone umfasst.

6. Harnkatheterprodukt nach einem der Ansprüche 1 bis 5, wobei das Hydratationsmedium ferner das Mittel umfasst.

7. Harnkatheterprodukt nach einem der Ansprüche 1 bis 6, ferner umfassend eine Verpackung.

## Revendications

1. Produit de cathéter urinaire, comprenant :
un cathéter ayant une tige de cathéter et un revêtement hydrophile sur la tige de cathéter,
dans lequel le revêtement hydrophile devient lubrifiant lorsqu'il est mouillé ; et
dans lequel le revêtement hydrophile comprend un agent qui entrave les structures de motilité de bactéries, dans lequel ledit agent comprend la punicalagine ;
dans lequel le produit de cathéter comporte en outre un milieu d'hydratation pour hydrater le revêtement hydrophile.

2. Produit de cathéter urinaire selon la revendication 1, dans lequel le milieu d'hydratation comprend de l'eau ou une solution saline.

3. Produit de cathéter urinaire selon la revendication 1 ou 2, dans lequel le cathéter comprend une aide à l'introduction.

4. Produit de cathéter urinaire selon l'une quelconque des revendications 1 à 3, dans lequel un manchon est fixé à l'aide à l'introduction, dans lequel le manchon entoure le cathéter.

5. Produit de cathéter urinaire selon l'une quelconque des revendications 1 à 4, dans lequel l'agent comprend en outre des acides gras à chaîne ramifiée, des hydroxyindoles, des dérivés de naphtalène, des analogues de quinazoline-2,4-diamino, des hydrazones de salicylaldéhyde, des anilines de salicylidène et/ou des thiazolidinones.

6. Produit de cathéter urinaire selon l'une quelconque des revendications 1 à 5, dans lequel le milieu d'hydratation comprend en outre l'agent.

7. Produit de cathéter urinaire selon l'une quelconque des revendications 1 à 6, comprenant en outre un emballage.
